# EUROPEAN PATENT APPLICATION

(11) **EP 1 403 638 A1**
(43) Date of publication of application: **31.03.2004**
(21) Application number: 02021413.6
(22) Date of filing: 25.09.2002
(51) Int. Cl.: G01N 33/48

(54) **Molecular methods for diagnosing interstitial lung diseases**

(71) Applicant: Mondobiotech SA, 6925 Gentilino (CH)
(72) Inventor: Bevec, Dorian, Dr., 6925 Gentilino (CH)
(74) Representative: Salgo, Reinhold Caspar, Dr.

(57) **Abstract**

The present invention relates to molecular methods diagnosing interstitial lung diseases using arrays of candidate polynucleotides and to methods useful in molecular evaluation of the efficacy of a drug applied to a person in need suffering from an interstitial lung disease by gene expression profiling images.

## Description

The present invention relates to molecular methods for diagnosing interstitial lung diseases using arrays of candidate polynucleotides and to screening assays for evaluating the efficacy of medicaments using gene expression profiling images from human materials.

With the completion of the Human Genome Project sequencing, biomedical research is being revolutionised by the ability to carry out investigations on a genome wide scale. Gene expression technology is gaining increasingly widespread use as a means to determine the expression of potentially all human genes at the level of messenger RNA. Gene specific sequences (oligo-, polynucleotides or cDNAs) are immobilised on arrays, hybridised with complex probes represented by a mixture of cDNAs of respective samples from human biopsies and other human materials (reversely transcribed from messenger RNA), and labelled with different dyes. Hybridised slides are analysed with sensitive scanning methodologies. Chips containing gene sequences deliver fast an excellent overview of the expression pattern of the biopsy sample.
Thus, the genome-wide gene expression analysis provides new insights into causes of disease, and elucidates the undiscovered biological variations between seemingly similar diseases, leading to a new classification system valuable in accurate diagnosis.

Important applications include:
(a) development of a more global understanding of the gene expression profiles that contribute to disease processes;
(b) discovery of new diagnostic and prognostic indicators and biomarkers of therapeutic response;
(c) identification and validation of new molecular targets for drug development;
(d) provision of an improved understanding of the molecular mode of action during lead identification and optimisation;
(e) designing more specific and efficient treatment strategies;
(f) prediction of potential side-effects during development and toxicology studies;
(g) confirmation of molecular modes of action during hypothesis-testing clinical trials;
(h) identification of genes involved in conferring drug sensitivity and resistance;
(i) prediction of patients most likely to benefit from the drug and use in general pharmacogenomic studies.

As a result of further technological improvements, the use of array-based Genechips will become an essential and routine tool for biomedical research and clinical intervention in identifying the disease signature genes, individual gene-expression patterns for purposes of patient-oriented therapy, and in establishing a method for predicting efficacy of drugs for individual patients, given the functional complexity of most diseases, especially those involving fibroproliferative processes. Considering the fact that inflammatory diseases reflect changes in the activity of certain genes and/or gene clusters, the molecular assessment will allow for a higher specificity and efficacy of medical treatments.
To achieve this goal, new molecular markers and markers of progression are needed for improved staging and for better assessment of diagnosis and treatment of inflammatory diseases. Gene expression profiling techniques offer the opportunity to discover such pathophysiologically relevant markers of disease.

It is now becoming state of the art to investigate disease signature gene expressions as demonstrated for a variety of diseases:
J Neurosci 2002 Apr 1;22(7):2718-29
   Gene expression profiling reveals alterations of specific metabolic pathways in **schizophrenia.**
   Middleton FA et al.
J Natl Cancer Inst 2002 Apr 3;94(7):513-521
   Osteopontin Identified as Lead Marker of **Colon Cancer** Progression, Using Pooled Sample Expression Profiling.
   Agrawal D et al.
Blood 2002 Mar 15;99(6):2037-44
   Gene expression profiling identifies significant differences between the molecular phenotypes of bone marrow-derived and circulating human CD34(+) **hematopoietic stem cells**.
   Steidl U et al.
Invest Ophthalmol Vis Sci 2002 Mar;43(3):603-7
   Gene expression profile of native human **retinal pigment epithelium.**
   Buraczynska M et al.
Cancer Res 2002 Feb 15;62(4):1184-90
   Molecular classification of **head and neck squamous cell carcinoma** using cDNA microarrays.
   Belbin TJ et al.
Oncogene 2002 Feb 21;21(9):1346-58
   Identification of genes regulated by **dexamethasone in multiple myeloma cells** using oligonucleotide arrays.
   Chauhan D et al.
Genome Res 2002 Feb;12(2):281-91
   In vivo regulation of human **skeletal muscle** gene expression **by thyroid hormone.**
   Clement K et al.
Cancer Res 2002 Feb 1;62(3):819-26
   Relationships and differentially expressed genes among **pancreatic cancers** examined by large-scale serial analysis of gene expression.
   Ryu B et al.
J Vasc Surg 2002 Feb;35(2):346-55
   Differential gene expression in human **abdominal aorta:** aneurysmal versus occlusive disease.
   Armstrong PJ et al.
Lancet 2002 Jan 12;359(9301):131-2
   Identification of high risk **breast-cancer** patients by gene expression profiling.
   Ahr A et al.
Nature 2002 Jan 31;415(6871):530-6
   Gene expression profiling predicts clinical outcome of **breast cancer**.
   van 't Veer LJ et al.
Oncogene 2002 Jan 17;21(3):475-8
   Global gene expression profiling in **Barrett's esophagus and esophageal cancer:** a comparative analysis using cDNA microarrays.
   Selaru FM et al.
Mol Carcinog 2002 Jan;33(1):25-35
   Gene expression analysis of **prostate cancers.**
   Luo JH et al.
Nature 2002 Jan 24;415(6870):436-42
   Prediction of central **nervous system embryonal tumour** outcome based on gene expression.
   Pomeroy SL et al.
Arch Ophthalmol 2001 Nov;119(11):1629-34
   Microarray analysis of gene expression in human **donor corneas.**
   Jun AS et al.
Ann Surg 2001 Dec;234(6):769-78; discussion 778-9
   Identification of disease-specific genes in **chronic pancreatitis** using DNA array technology.
   Friess H et al.
FASEB J 2002 Jan;16(1):61-71
   Gene profiling reveals unknown enhancing and suppressive **actions of glucocorticoids on immune cells.**
   Galon J et al.
Cancer Res 2002 Jan 1;62(1):233-40
   Global gene expression analysis of **gastric cancer** by oligonucleotide microarrays.
   Hippo Y et al.
Nat Med 2002 Jan;8(1):68-74
   Diffuse large **B-cell lymphoma** outcome prediction by gene-expression profiling and supervised machine learning.
   Shipp MA et al.
DNA Cell Biol 2001 Nov;20(11):683-95
   A gene expression profile of **Alzheimer's disease.**
   Loring JF et al.
Mol Carcinog 2002 Feb;33(2):113-24
   Analysis of differentially expressed genes in **hepatocellular carcinoma** using cDNA arrays.
   Goldenberg D et al.
J Dent Res 2002 Feb;81 (2):89-97
   DNA hybridization arrays for gene expression analysis of human **oral cancer.**
   Todd R, Wong DT
Inflamm Bowel Dis 2002 Mar;8(2):140-57
   Application of genome-wide gene expression profiling by high-density DNA arrays to the treatment and study of **inflammatory bowel disease.**
   Warner EE, Dieckgraefe BK.
Invest Ophthalmol Vis Sci 2002 Mar;43(3):603-7
   Gene expression profile of native human **retinal pigment epithelium.**
   Buraczynska M et al.
Adv Cancer Res 2002;84:1-34
   Gene expression in inherited **breast cancer.**
   Hedenfak IA et al.
Pharmacogenomics J 2001;1(4):272-87
   Molecular classification of **psoriasis** disease-associated genes through pharmacogenomic expression profiling.
   Oestreicher JL et al.

In addition, a significant number of patent applications are addressing the described molecular techniques in medical indications, for example:

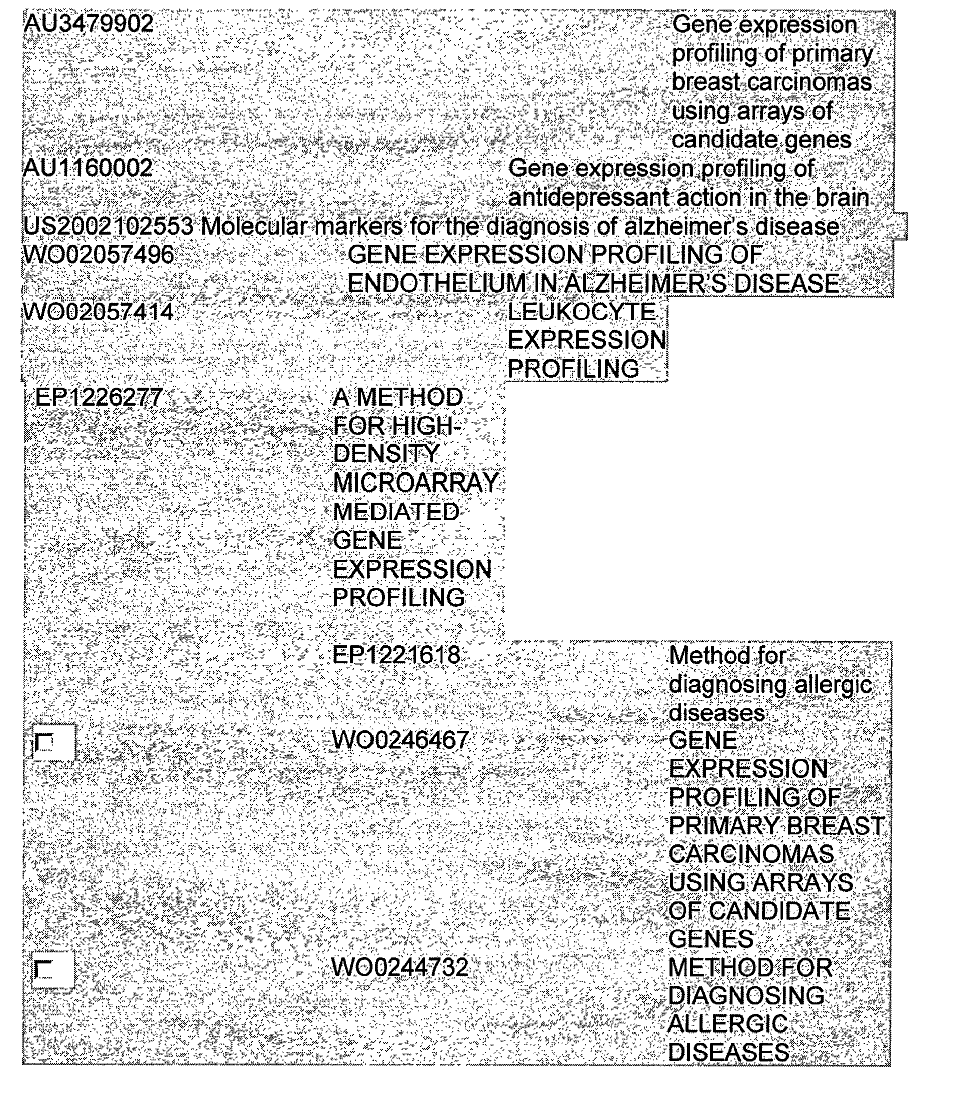

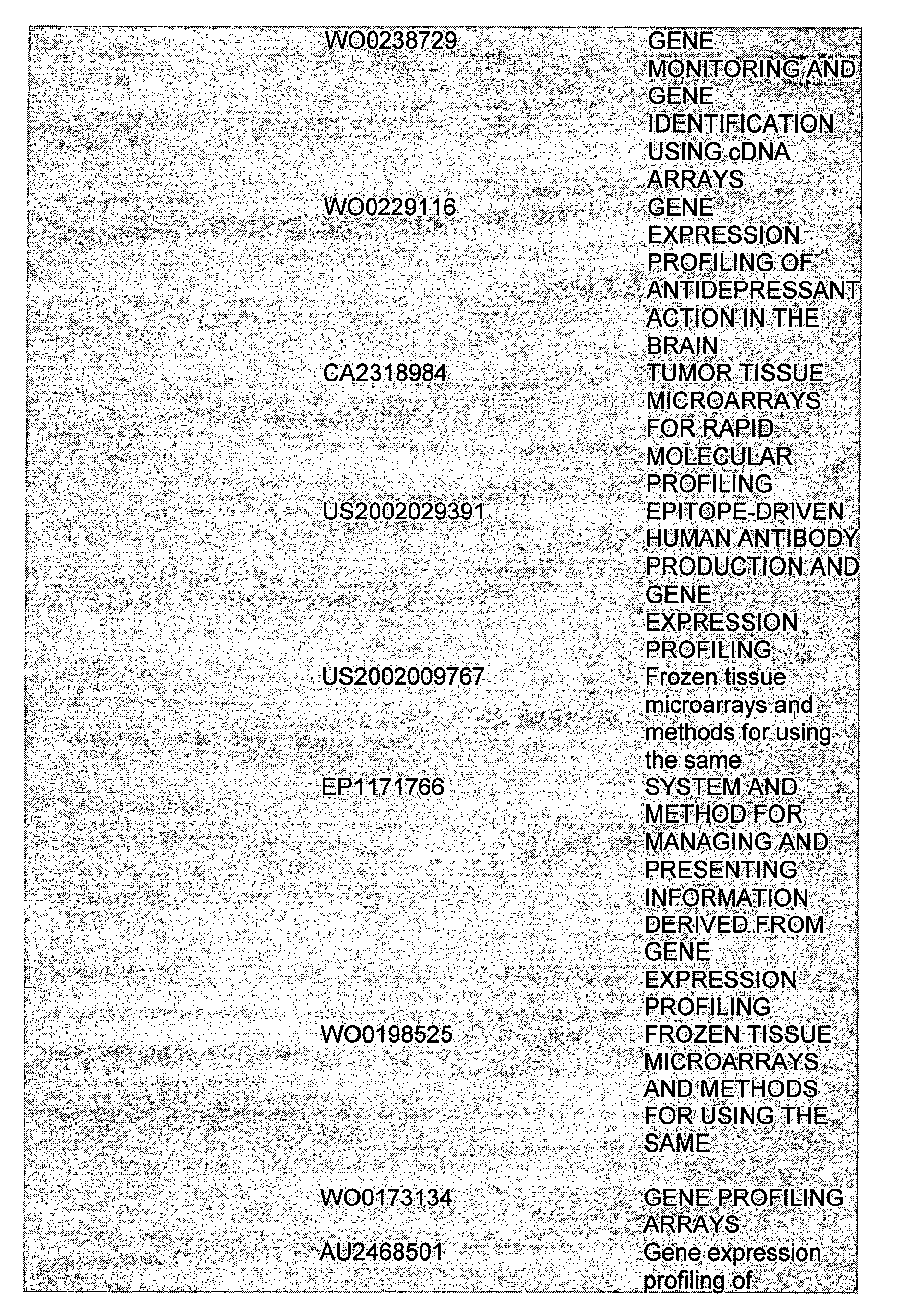

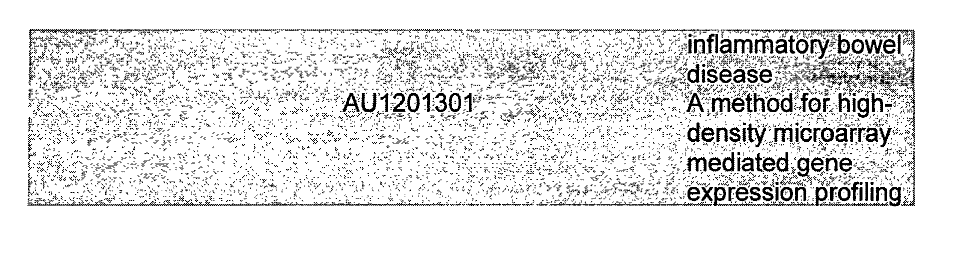

### Interstitial lung diseases

Reliable diagnosis of is of critical importance for disease management, research, epidemiology and development of specific therapies.
Interstitial lung diseases (ILD) are a heterogeneic group of chronic inflammatory reactions of the lung. Different forms of ILD are known which comprise, for example, of idiopathic pulmonary fibrosis (IPF), hypersensivity pneumonitis, sarcoidosis, scleroderma, Systemic Lupus Erythematosus, Rheumatoid Arthritis, Churg-Strauss syndrome, Wegener's granulomatosis, and Goodpasture Syndrome. This process is characterized by a combination of injury and exaggerated but futile attempts of tissue repair that transforms the regular maintenance of cellular growth into progressive development of scars. Characteristic features of this reaction are: repeated damage; intensified proteolytic activity and change in the composition of extracellular matrix (ECM) components. This combination leads to a shifted cellular immune response and a relentless induction of mesenchymal growth.
Cytokines, such as tumor necrosis factor-α, and mediators of growth, such as transforming growth factor β₁ (TGFβ₁), have long been implicated in this process. Of these mediators, TGFβ₁ has probably become the most important one, due to its strong activity to stimulate mesenchymal growth and its ability to modulate cellular immune functions. TGF-β₁ is known to cause severe pulmonary fibrosis when overexpressed in animal models, and a significant overexpression of this mediator is found in human pulmonary fibrosis. The immunomodulatory action of TGF-β₁ is well-known. It includes the inhibition of interferon gamma release, the suppression of interferon gamma-dependent immune reactions, and the induction of immunosuppressive CD8+ lymphocytes. Indeed, modulation of cellular immunity in patients with progressive fibrosis has been observed for years, even in forms, which clearly represent *different* mechanisms of initial damage. Recent investigations have demonstrated that progressive scarring in idiopathic pulmonary fibrosis is accompanied by a shift of the cytokine balance in T lymphocytes that favors the formation of the so-called T helper type 2 (Th2) reaction. This reaction is characterized by an increase of 'Th2' cytokines, such as interleukin (IL)-4, IL-10 and IL-13, and a reduction or even a complete loss of interferon gamma, the main mediator of the T helper type 1 reaction. In bleomycin-induced lung fibrosis, transcription of the interferon gamma gene decreases from day 7 onwards and is no longer detectable. on day 28. Reciprocally, transcription of IL-13, which also has fibrogenic activity, begins to increase on day 7.

In contrast to the chronic inflammatory reaction in fibrosis, acute inflammation of the pulmonary interstitium as a result of infection with *M. pneumoniae* is characterized by a simultaneous transcription of both Th1 and Th2 cytokine genes, i.e. IL-12 and interferon gamma, and IL-4. In addition, this reaction includes an increased transcription of TGF-β₁, probably as a sign of activated tissue repair.

Unfortunately, as a result of the usually late recognition of idiopathic pulmonary fibrosis, the early cellular events in this disease are virtually impossible to assess. However, analysis of mRNA accumulation for IL-4, interferon gamma, and TGF-β₁ in a patient with familial idiopathic pulmonary fibrosis, with symptoms of breathlessness on maximum exertion of less than a year, already demonstrates the shift of the immune balance and the intense activation of transcription of TGF-β₁. In the group of patients with idiopathic pulmonary fibrosis, we found that the amount of mRNA accumulation of the TGF-β₁ gene was seven fold higher than that in normal lungs. In conclusion, our observations clearly demonstrate features of pathologically intensified repair mechanisms.

The intensity of repair is directly influenced by mechanisms of inflammation causing intensified turnover of both ECM and cellular components. As a result, chronic inflammation, usually induced by various infective agents, aggravates pathologic tissue repair.

Even if the causative agents were known, at present organ fibrosis cannot be sufficiently treated with any medication. Millions of people are dying from slow destruction of vital organ systems owing to pathological restructuring of functional organ tissue. This relentless process is known as fibrosis or tissue remodeling that is primarily due to fibroproliferative mechanisms. The only remedy is organ transplantation, which is associated with numerous costly complications.
The fibroproliferative reaction concerns all organs of the body. In the gas-exchanging lung tissue it is known as lung fibrosis, in the bronchi it is known as bronchial asthma, in the liver as cirrhosis, in the kidney as glomerulosclerosis, in the general circulation as arteriosclerosis and coronary artery sclerosis, and in the pulmonary circulation as pulmonary hypertension.
These conditions are collectively known as fibroproliferative diseases. In fibrosis, healthy tissue is progressively replaced by components of the connective and supporting tissue of the human body. This process is based on the pathologically accelerated growth rate of tissue cells, which would normally accomplish regular wound healing. Thus, fibroproliferative diseases may be defined as uncontrollably accelerated wound healing. In fibrosis, the replacement of functional organ tissue continues until complete loss of organ function occurs. The currently available modes of diagnosis and treatment for all fibroproliferative diseases are inadequate.

In order to optimize the beneficial, antifibrotic effect of Interferon-γ, a reliable diagnosis is key. To date, more than 150 different mechanisms are known which can damage the lung and cause fibroproliferative wound healing. These include chronic infections, exposure to organic and inorganic dusts, various drugs, and mechanisms of autoimmunity. Nonetheless, the fibrotic process is an entity of itself. It is, therefore, mandatory to dissect out the fibrotic response from the accompanying inflammatory response. For example, minor inflammation may result in a dramatic progression of fibrosis, as well as in a high intensity of inflammatory activity, reflecting the "need" of an inflammatory "input" in some cases, and the nearly complete "autoregulation" of fibrosis in others. Chronic viral infections represent probably the most common processes leading to progressive fibrosis "despite" immunosuppressive treatment. These facts provide a rough overview over the immense diagnostic problems in interstitial lung diseases. The same holds true for chronic bacterial and fungal infections, with or without chronic gastric reflux that may cause chronic inflammation of the terminal bronchi that will directly influence the chronic wound healing process in the surrounding alveoli.

Therefore, a molecular approach that defines and recognizes important mechanisms of damage, inflammation and wound healing will be able to provide a much better tool for diagnosis and treatment of these multifunctional diseases. The data achieved by applying this diagnostic chip will not only help to diagnose the disease but will provide additional insight into the disease process and accelerate the development of specific medications of lung fibrosis.

The design of new multigenic diagnostic systems is of critical importance for future management of multigenic diseases like ILD. These systems bear the possibility to define subclasses of the diseases, which allow better treatment and design of new drugs, to define disease risk-groups, to define monitoring of drugs in patients, to identify patients which may develop drug resistance, and to identify the disease-causing genes.

Interferon gamma-1b was shown to be the first applicable anti-fibrotic drug. As any endogenous substance, interferon gamma-1b has pleiotropic effects dependent on the existing conditions within the body. In case of an exaggerated, yet non- or low-inflammatory wound healing, such as in idiopathic pulmonary fibrosis, the drug exerts powerful highly beneficial anti-fibrotic properties. However, in the presence of accompanying infections, such as chronic viral or fungal infections, the drug will add to the inflammatory process and even reinforce, by intensifying tissue repair, the fibrotic process.

Thus, for a safe and effective use of the drug, it is necessary to measure the state of tissue response prior to application of the drug. Fortunately, the absence of the transcription of the gene for interferon gamma in the presence of intensified transcription of factors of wound healing may serve as the first discriminator between fibrosis as a result of deregulated wound healing itself, versus fibrosis as a result of intensified inflammation. The first attempts to analyze gene expression patterns in pulmonary fibrosis in animal models demonstrate the power of this tool to discriminate between the fibrotic process itself and accompanying mechanisms: Kaminski et al., Proc Natl Acad Sci U S A 2000 Feb 15;97(4):1778-83 Global analysis of gene expression in pulmonary fibrosis reveals distinct programs regulating lung inflammation and fibrosis; Katsuma et al., Biochem Biophys Res Commun 2001 Nov 9;288(4):747-51 Molecular monitoring of bleomycin-induced pulmonary fibrosis by cDNA microarray-based gene expression profiling. An approach that combines transcription analysis with antifibrotic effectiveness under clinical conditions may possibly provide the essential step for the first biologically based treatment of these multifunctional diseases.

In the context of this disclosure, a number of terms shall be utilized.
The term "polynucleotide" refers to a polymer of RNA or DNA that is single-stranded, optionally containing synthetic, non-natural or altered nucleotide bases. A polynucleotide in the form of a polymer of DNA may be comprised of one or more segments of cDNA, genomic DNA or synthetic DNA.
The term "subsequence" refers to a sequence of nucleic acids that comprises a part of a longer sequence of nucleic acids.
The term "immobilized on a support" means bound directly or indirectly thereto including attachment by covalent binding, hydrogen bonding, ionic interaction, hydrophobic interaction or otherwise.

An aspect of the invention relates to the use of polynucleotide arrays, which allows to quantitatively study mRNA expression levels of selected candidate genes in human biopsies.

Polynucleotide or DNA arrays consist of large numbers of DNA molecules spotted in a systematic order on a solid support or substrate such as a nylon membrane, glass slide, glass beads or a silicon chip. Depending on the size of each DNA spot on the array, DNA arrays can be categorized as microarrays (each DNA spot has a diameter less than 250 microns) and macroarrays (spot diameter is grater than 300 microns). When the solid substrate used is small in size, arrays are also referred to as DNA chips. Depending on the spotting technique used, the number of spots on a glass microarray can range from hundreds to tens of thousands.

DNA microarrays have serve a variety of purposes, including, gene expression profiling, de novo gene sequencing, gene mutation analysis, gene mapping and genotyping. cDNA microarrays are printed with distinct cDNA clones isolated from cDNA libraries. Therefore, each spot represents an expressed gene, since it is derived from a distinct mRNA.

Typically, a method of monitoring gene expression involves providing
(1) a pool of sample polynucleotides comprising RNA transcripts of target genes or nucleic acids derived from the RNA transcripts;
(2) hybridizing the sample polynucleotides or nucleic acids derived from the RNA transcripts to an array of probes (for example, polynucleotides obtained from a polynucleotide library including control probes, and
(3) detecting the hybridized double- calculating/quantifying a relative expression (transcription) level. stranded polynucleotides/nucleic acids. The label used to label polynucleotide samples is selected from the group consisting of radioactive, colorimetric, enzymatic, molecular amplification, bioluminescent or fluorescent label.

The invention relates also to any polynucleotide library as previously described wherein said polynucleotides are immobilized on a solid support in order to form a polynucleotide array.

Preferably the support is selected from the group consisting of a nylon membrane, glass slide, glass beads, or a silicon chip.
The invention relates to a polynucleotide library useful in the molecular characterization of a fibrotic interstitial lung disease like Idiopathic Pulmonary Fibrosis (IPF), the library including a pool of polynucleotide sequences or subsequences thereof wherein the sequences or subsequences are either underexpressed or overexpressed in IPF diseased cells.

Preferably, a set of sequences is selected from oligo- or polynucleotide probes having a sequence defined by, or correlated to, or derived from the group of genes consisting of candidate genes indicated in the following list, representing increased gene expression levels of IPF cells versus cells from patients with a granulomatous, non-fibrotic lung disorder:
PTH-responsive osteosarcoma B1 protein, AF095771.1
matrix associated, actin dependent regulator of chromatin, subfamily f, member 1, AF231056.1
deleted in lung and esophageal cancer 1 (DLEC1), NM_007337.1
major histocompatibility complex, class II, DQ beta 1, AW276186
SB classII histocompatibility antigen alpha-chain, AI128225
mucin 4, tracheobronchial, AJ242547.1
forkhead box J1 (FOXJ1), U69537.1
hypothetical protein FLJ21616, NM_024567.1
neuronal specific transcription factor DAT1, AF258348.1
hematopoietic PBX-interacting protein, BF344265
proline oxidase homolog, AA074145
mucin 5, subtype B, tracheobronchial, AI697108
golgi membrane protein GP73, AF236056.1
ATP citrate lyase, U18197.1
NG22 protein, NM_025257.1
cDNA DKFZp434A2322, AL137706.1
hepatocyte nuclear factor 3, alpha, U39840.1
major histocompatibility complex, class II, DQ alpha 1, X00452.1
myosin regulatory light chain 2, smooth muscle isoform, J02854.1
plexin B1, AV693216
pyruvate kinase, muscle, BC000481.1
tetraspanin TM4-C, AF133425.1
insulin-like growth factor binding protein 2 (36kD), BC004312.1
FLJ13945 fis, clone Y79AA1000969, AU160041
hypothetical protein DKFZp586M1120, NM_031294.1
CD24 signal transducer, L33930
hypothetical protein FLJ23571, NM_025111.1
glutathione S-transferase M2 (muscle), M63509.1
cadherin 1, type 1, E-cadherin (epithelial), L08599.1
NTT5 protein, AF265578.1
lipocalin 2 (oncogene 24p3), NM_005564.1
myotonic dystrophy kinase (DM kinase), L08835
uncoupling protein 2 (mitochondrial, proton carrier), U76367.1
dynein intermediate chain 2, NM_023036.1
discoidin receptor tyrosine kinase isoform b, discoidin domain receptor family, member 1, NM_001954.2
sperm associated antigen 6, AF079363.1
hypothetical protein FLJ23049, NM_024687.1
nasopharyngeal epithelium specific protein 1, AF094758.1
nuclear receptor subfamily 4, group A, member 2, NM_006186.1
hypothetical protein FLJ22215, BC003543.1
non-specific cross reacting antigen, M18728.1
amylase, alpha 1A; salivary (AMY1A), NM_004038.1
carcinoembryonic antigen-related cell adhesion molecule 6 (non-specific cross reacting antigen), BC005008.1
glutathione S-transferase subunit 4 (EC 2.5.1.18), X08020.1
SH3-containing protein SH3GLB2, AF257319.1
KDEL (Lys-Asp-Glu-Leu) endoplasmic reticulum protein retention receptor 1, NM_006801.1
anterior gradient 2 (Xenepus laevis) homolog, AF038451.1
sv7-MUC4 apomucin, mucin 4, tracheobronchial, AJ242547.1
stratifin, BC000329.1
connective tissue growth factor, M92934.1
cytochrome P450-IIB (hIIB3), M29873.1
filamin A, alpha (actin-binding protein-280), AW051856
membrane glycoprotein LIG-1, AB050468.1
E74-like factor 3 (ets domain transcription factor, epithelial-specific ), U73844.1
elastin (supravalvular aortic stenosis, Williams-Beuren syndrome), M36860.1
ephrin receptor EPHA3, AF213459.1
fibrillin 1 (Marfan syndrome), L13923.1
discs, large (Drosophila) homolog 1, U13896.1
lysyl oxidase-like 1 (LOXL1), L21186.1
calumenin, U67280.1
male germ cell-associated kinase, NM_005906.2
plectin 1, intermediate filament binding protein, 500kD, Z54367
peroxisome biogenesis factor 1, AF026086.1
mast cell tryptase beta III, AF099143
ataxia-telangiectasia group D-associated protein, AF230388.1
hypothetical protein FLJ10921, NM_018272.1
biglycan, BC002416.1
BLu protein, AC002481
CGI-92 protein, AF151850.1
adaptor-related protein complex 1, mu 2 subunit, BC003387.1
keratin 15, BC002641.1
B7 protein, U72508.1
S100 calcium-binding protein A2, BC002829.1
MUF1 protein, BC004953.1
   cholesterol 25-hydroxylase, AF059214.1
cytidine monophosphate-N-acetylneuraminic acid hydroxylase (CMP-N-acetylneuraminate monooxygenase), AF074480.1
neuropilin 2, AF022859.1
Fas-interacting serinethreonine kinase 3, homeodomain-interacting protein kinase 3, AF305239.1
a disintegrin and metalloproteinase domain 28 (ADAM28), transcript variant 2, AF137334.1
cDNA DKFZp434A119 , AW663632
complement component 6, J05064.1
cytokeratin 17, Z19574
wingless-type MMTV integration site family, member 5A, AI968085
matrix metalloproteinase 7 (matrilysin, uterine), BC003635.1
leiomodin 1 (smooth muscle), NM_012134.1
Cip1-interacting zinc finger protein, AB030835.1
cyclin-dependent kinase inhibitor 1A (p21, Cip1), BC000275.1
integrin, alpha 7, AF032108.1
DKFZP586G011 protein, BG289527
fatty acid binding protein 6, ileal (gastrotropin), U19869.1
glutathione S-transferase M4, M96234.1
Ras-related associated with diabetes, L24564.1
claudin 3, AB000714.1
matrix metalloproteinase 10 (stromelysin 2), BC002591.1
fibulin 2, NM_001998.1
serine threonine kinase 11 (STK11), AF035625
transforming growth factor β₁
wherein each oligo- or polynucleotide probe is obtainable on the basis of structural information mediated by the sequence data provided by the respective accession number set out for each candidate gene.

Yet another set of sequences is selected from oligo- or polynucleotide probes having a sequence defined by, or correlated to, or derived from the group of genes consisting of candidate genes indicated in the following list, representing decreased gene expression levels of IPF cells versus cells from patients with a granulomatous, non-fibrotic lung disorder:
sorting nexin 10 (SNX10), AF121860.1
phospholipase A2, group IIA (platelets, synovial fluid), M22430.1
hemoglobin alpha-1 globin chain (HBA1), AF349571.1
macrophage scavenger receptor 1, AI299239
surfactant, pulmonary-associated protein C, BC005913.1
disintegrin protease (M12.219), NM_014479.1
retinol-binding protein 4, interstitial, AF119868.1
major histocompatibility complex, class II, DR beta 3, M27635.1
adipose differentiation-related protein, BC005127.1
hemoglobin, delta, NM_000519.2
beta-2-microglobulin, AW188940
hemoglobin, alpha 2, BC005931.1
protein C receptor, endothelial (EPCR), L35545.1
thymosin, beta 10, M92381.1
apolipoprotein C-I, W79394
hypothetical protein, AL133067.1
proteoglycan 4, (megakaryocyte stimulating factor, articular superficial zone protein), U70136.1
tetranectin (plasminogen-binding protein), NM_003278.1
platelet factor 4, M25897.1
tissue factor pathway inhibitor 2, BC005330.1
fatty acid binding protein 4, adipocyte, BC003672.1
cathepsin B (CTSB), M14221.1
transmembrane 4 superfamily member 1, M90657.1
MHC class I HLA-B51 major histocompatibility complex, class I, E, M31183.1 endothelial PAS domain protein 1, U51626.1
Apo-2 ligand tumor necrosis factor (ligand) superfamily, member 10, U37518.1 haptoglobin-related protein, NM_020995.1
Rho guanine exchange factor (GEF) 12, AF119898.1
major histocompatibility complex, class II, DR beta 5, M11867.1
interferon, gamma-inducible protein 30, AF097362.1
ferritin, light polypeptide, BG537190
prosaposin (variant Gaucher disease and variant metachromatic leukodystrophy), BC004275.1
calcium-binding protein A4 (calvasculin, metastasin,), NM_002961.2
hemoglobin, beta, M25079.1
CDW52 antigen (CAMPATH-1 antigen), BC000644.1
aldehyde dehydrogenase 1 family, member A2, AB015226.1
cathepsin Z, AF032906.1
MHC HLA-B39 major histocompatibility complex, class I, B, L37880.1
major histocompatibility complex, class II, DQ alpha 1, M33906.1
fibroblast growth factor 9 (glia-activating factor), D14838.1
hemoglobin, alpha 2, AF097635.1
transferrin receptor (p90, CD71), BC001188.1
complement component 3 (C3), K02765.1
cDNA DKFZp564D066, AL050025.1
complement component 1, q subcomponent, beta polypeptide, NM_000491.2
small inducible cytokine subfamily A (Cys-Cys), member 18, pulmonary and activation-regulated, AB000221.1
reticulon 1, L10333.1
major histocompatibility complex, class II, DR beta 1, M33600.1
haptoglobin, L29394.1
acid phosphatase 5, tartrate resistant, J04430.1
cytochrome P450, subfamily XXVIIA (steroid 27-hydroxylase, cerebrotendinous xanthomatosis), polypeptide 1, M62401.1
CD36 antigen (collagen type I receptor, thrombospondin receptor), M24795.1
calbindin 2, (29kD, calretinin), NM_001740.2
alpha-2-HS-glycoprotein, BG538564
cDNA DKFZp564A132, AL049963.1
fibronectin 1, AF130095.1
phosphodiesterase 4C, cAMP-specific, NM_000923.1
transcription factor 7 (T-cell specific, HMG-box), NM_003202.1
found in inflammatory zone 3 (FIZZ3), AF323081.1
claudin 15, NM_014343.1
carboxypeptidase B1 (tissue), M81057.1
hypothetical protein FLJ14054, NM_024563.1
bone marrow stromal cell antigen 1, D21878.1
interleukin 7 receptor, M29696.1
procollagen C-endopeptidase enhancer 2, AF098269.1
calcium-binding protein A8 (calgranulin A), AW238654
cDNA DKFZp564D193, AL049252.1
major histocompatibility complex, class II, DP beta 1, J03041.1
human leukocyte antigen C alpha chain, major histocompatibility complex, class I, C, AK024836.1
BCM-like membrane protein precursor, AF144235.1
CD14 antigen, M86511.1
pulmonary surfactant protein (SP5), J03553
signal transducer and activator of transcription 1, 91 kD, BC002704.1
Wilms tumor 1 (WT1), transcript variant D, NM_024424.1
annexin A8, BC004376.1
macrophage receptor with collagenous structure, MARCO, AF035819.1
surfactant, pulmonary-associated protein A2, NM_006926.1
solute carrier family 6 (neurotransmitter transporter, serotonin), member 4, L05568.1
chitinase 1 (chitotriosidase), U29615.1
lung type-I cell membrane-associated glycoprotein, AU154455
fibronectin leucine rich transmembrane protein 2, AB007865.1
gamma-aminobutyric acid (GABA) A receptor, alpha 5, BF966183
hypothetical protein FLJ12983, NM_024856.1
sialophorin (gpL115, leukosialin, CD43), J04536.1
cerebellar degeneration-related protein (34kD), M16965.1
hydroxyacid oxidase 2 (long chain), hydroxy-delta-5-steroid dehydrogenase (3 beta-and steroid delta-isomerase 2), AL359553
interferon-gamma
wherein each oligo- or polynucleotide probe is obtainable on the basis of structural information mediated by the sequence data provided by the respective accession number set out for each candidate gene.

A third set of sequences is selected from oligo- or polynucleotide probes having a sequence defined by, or correlated to, or derived from the group of genes consisting of candidate genes indicated in the following list, representing increased gene expression levels of IPF cells versus cells from patients with a different fibrotic lung disorder EAA:
eukaryotic translation initiation factor 1A: AF000987.1
DEADH (Asp-Glu-Ala-AspHis) box polypeptide: AF000985.1
ribosomal protein S4: AF116711.1
ubiquitin specific protease 9: AF000986.2
SMC (mouse) homolog: U52191.1
myelin basic protein: L18865.1
S100 calcium-binding protein: NM_005980.1
Jagged2 (JAG2): AF003521.1
latent transforming growth factor beta binding protein 4: NM_003573.1
microtubule-associated protein, RPEB family, member 3: AB025186.1
Unknown (protein for MGC:2854): BC003629.1
clone=IMAGE-2406340: AI830563
AQP3 gene for aquaporine 3 (water channel): AB001325
cDNA DKFZp434A119
fenestrated-endothelial linked structure protein (FELS), PV1 protein (PLVAP): AF326591.1
LUNX protein; PLUNC (palate lung and nasal epithelium clone); tracheal epithelium enriched protein (LOC51297): AB024937.1
RAB, member of RAS oncogene family-like 2A: AF095350.1
chromosome 11 open reading frame 16: NM_020643.1
hypothetical protein FLJ23049: NM_024687.1
hypothetical protein FLJ11767: NM_024593.1
dynein, axonemal, intermediate polypeptide: AF091619.1
brain specific protein (LOC51673): AF132972.1
MUC4 apomucin, mucin 4, tracheobronchial: AJ242547.1
myotonin protein kinase (DM): M87313.1
cytokeratin 4: X07695.1
KIAA0362 gene, MCF.2 cell line derived transforming sequence-like: AB002360.1
cytokeratin 17: Z19574
LIM domain protein: BC003096.1
E74-like factor 3 (ets domain transcription factor, epithelial-specific) : U73844.1
fatty acid binding protein 6, ileal (gastrotropin): U19869.1
sperm associated antigen 6: AF079363.1
eyes absent (Drosophila) homolog 2: U71207.1
phosphatidic acid phosphatase type 2C: BC002806.1
epoxide hydrolase 2, cytoplasmic: AF233334.1
tubulin, beta, 2: BC002783.1
heat shock 105kD: D86956.1
villin 2 (ezrin): J05021.1
a disintegrin and metalloproteinase domain 28 (ADAM28), transcript variant 3: AF137335.1
deleted in lung and esophageal cancer 1: NM_007337.1
arachidonate 15-lipoxygenase: NM_001140.1
UDP glycosyltransferase 1 family, polypeptide A1: M57899.1
hypothetical protein PRO2834: AF119903.1
lectin, galactoside-binding, soluble, 7 (galectin 7): L07769.1
B7 protein: U72508.1
ephrin receptor EPHA3: AF213459.1
forkhead box J1: U69537.1
BLu protein: U70824.1
aldehyde dehydrogenase 3 family, member A1: BC004370.1
NG22 protein: NM_025257.1
small inducible cytokine subfamily A (Cys-Cys), member 14: NM_004166.1
cysteine-rich protein 1 (intestinal): BC002738.1
putative GTP-binding protein similar to RAYRAB1C: BC000566.1
integrin, beta 4: NM_000213.1
serine (or cysteine) proteinase inhibitor, clade B (ovalbumin), member 5 (SERPINB5): U04313.1
Ras-related associated with diabetes: L24564.1
hepatic leukemia factor: M95585.1
keratin 15: BC002641.1
nuclear receptor subfamily 4, group A, member 2: NM_006186.1
sialyltransferase: U14550.1
glutathione S-transferase M2 (muscle): M63509.1
hypothetical protein FLJ13110: NM_022912.1
S100 calcium-binding protein A2: NM_005978.2
collagen, type VII, alpha 1 (epidermolysis bullosa, dystrophic, dominant and recessive) : L02870.1
claudin 3: AB000714.1
insulin-like growth factor binding protein 6: BC003507.1
fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome): M80634.1
insulin-like growth factor 1 receptor: NM_000875.2
insulin-like growth factor binding protein 2 (36kD): M35410.1
ataxia-telangiectasia group D-associated protein: AF230388.1
keratin 5 (epidermolysis bullosa simplex, Dowling-MearaKobnerWeber-Cockayne types): M21389.1
Duffy blood group: U01839.1
wherein each oligo- or polynucleotide probe is obtainable on the basis of structural information mediated by the sequence data provided by the respective accession number set out for each candidate gene.

Next set of sequences is selected from oligo- or polynucleotide probes having a sequence defined by, or correlated to, or derived from the group of genes consisting of candidate genes indicated in the following list, representing decreased gene expression levels of IPF cells versus cells from patients with a different fibrotic lung disorder Exogeneous Allergic Alveolitis:
CLONE=IMAGE:1032795=Hs.83623 nuclear receptor subfamily 1, group I, member 3 small inducible cytokine subfamily C, member 2: NM_003175.1
hypothetical protein similar to swine acylneuraminate lyase: NM_030769.1
fibrinogen, gamma polypeptide: AF118092.1
thrombospondin 1: NM_003246.1
SAM domain, SH3 domain and nuclear localisation signals, 1: AF222927.1
chitinase 3-like 1 (cartilage glycoprotein-39): M80927.1
cathepsin Z: AF032906.1
CLONE=IMAGE:3579023 collagen, type XIV, alpha 1 (undulin)
chloride intracellular channel 2: NM_001289.2
monokine induced by gamma interferon: NM_002416.1
KIAA0433 protein: NM_015216.1
tumor necrosis factor, alpha-induced protein 6: NM_007115.1
signal transducer and activator of transcription 1, 91 kD: BC002704.1
thrombospondin 2: L12350.1
collagen, type IV, alpha 3 (Goodpasture antigen): NM_000091.1
integrin, beta-like 1 (with EGF-like repeat domains): AF072752.1
diubiquitin: NM_006398.1
protease, cysteine, 1 (legumain): D55696.1
cytochrome P450, subfamily I (dioxin-inducible), polypeptide 1 (glaucoma 3, primary infantile): U03688.1
integrin, alpha 1: X68742.1
GABA-B receptor, G protein-coupled receptor 51: AF056085.1
KIAA1199 protein: AB033025.1
collagen, type V, alpha 2: NM_000393.1
interleukin 13 receptor, alpha 2: U70981.1
translocase of inner mitochondrial membrane 8 (yeast) homolog A: BC005236.1
steroid sulfatase (microsomal), arylsulfatase C, isozyme S: M16505.1
CLONE=IMAGE:1982571 ATPase, H+ transporting, lysosomal (vacuolar proton pump) 9kD
proteoglycan 4, (megakaryocyte stimulating factor, articular superficial zone protein): U70136.1
nidogen (enactin): M30269.1
KIAA1598 protein: AU157109
   vascular cell adhesion molecule 1: M60335.1
guanylate binding protein 1, interferon-inducible, 67kD: BC002666.1
cDNA DKFZp586E1124
apolipoprotein H (beta-2-glycoprotein I): M62839.1
ribosomal protein L37a: BE857772
cDNA DKFZp564A132
cathepsin S: M86553.1
a disintegrin and metalloproteinase domain 9 (meltrin gamma) (ADAM9): U41766.1
zinc finger protein 331: AF272148.1
lysosomal-associated membrane protein 2: J04183.1
carboxypeptidase M: NM_001874.1
collagen, type I, alpha 2: NM_000089.1
P311 protein: U36189.1
KIAA0372 gene product: AB002370.1
Human T cell-specific protein RANTES: M21121
interferon-gamma-inducible indoleamine 2,3-dioxygenase (IDO): M34455.1
hypothetical protein FLJ10430: NM_018092.1
transcription factor ISGF-3: M97935
interleukin 1 receptor-like 1 (IL1RL1): NM_003856.1
putative alpha chemokine (H174), small inducible cytokine subfamily B (Cys-X-Cys), member 11: AF002985.1
small inducible cytokine subfamily B (Cys-X-Cys), member 10: NM_001565.1
mesoderm specific transcript (mouse) homolog: BC002413.1
carboxypeptidase B-like protein: AB011969.1
CD2 antigen (p50), sheep red blood cell receptor: M16445.
interferon, alpha-inducible protein (clone IFI-6-16): NM_022872.1
RAS guanyl releasing protein 1 (calcium and DAG-regulated): AF081195.1
lipase, endothelial: AF118767.1
fatty-acid-Coenzyme A ligase, long-chain 4: NM_022977.1
klotho: AB005142.1
chondroitin sulfate proteoglycan 2 (versican): NM_004385.1
hypothetical protein, expressed in osteoblast: AB000115.1
CD14 antigen: M86511.1
CGI-83 protein: BC000878.1
leucine aminopeptidase: AF061738.1
UDP-Gal:betaGlcNAc beta 1,3-galactosyltransferase, polypeptide 3: AB050855.1
protocadherin alpha 12: AF152308.1
neuroglycan C: AF059274
neuroligin: AI338338
HSPC156 protein: AF161505.1
hypothetical protein FLJ13310: NM_025118.1
eosinophil chemotactic cytokine (TSA1902): AB025008.1
aminopeptidase: AF191545.1
semaphorin sem2: AB029496.1
protocadherin 12: AF231025.1
X transporter protein 3: NM_020208.1
transmembrane 4 superfamily member (tetraspan NET-2): AF124522.1
hypothetical protein FLJ10970: NM_018286.1
perforin 1 (pore forming protein): M28393.1
natural killer cell group 7 sequence: NM_005601.1
hypothetical protein DKFZp761 N09121: BF435376 integrin, alpha 4 (antigen CD49D, alpha 4 subunit of VLA-4 receptor): BG532690
chitinase 3-like 2: U58515.1
FYN oncogene: N20923
relaxin 1 (H1): BC005956.1
hydroxyprostaglandin dehydrogenase 15-(NAD): U63296.1
sulfotransferase family, cytosolic, 1C, member 1: AF186254.1
TGF-b superfamily receptor type I: L17075.1
granzyme B (granzyme 2, cytotoxic T-lymphocyte-associated serine esterase 1): M36118.1
cystatin F (leukocystatin): AF031824.1
regulator of G protein signaling-Z (RGSZ1): AF060877.2
clone MGC:12387: M16942.1
zinc-alpha2-glycoprotein: D90427.1
BCG induced integral membrane protein BIGMo-103: AB040120.1
nectin-like protein 2 (NECL2): AF132811.1
CD209 antigen-like: AB015629.1
solute carrier family 6 (neurotransmitter transporter, serotonin), member 4: L05568.1
MAD (mothers against decapentaplegic, Drosophila) homolog 6: U59914.1
latrophilin: AF104939.1
platelet factor 4: M25897.1
calcitonin receptor-like: L76380.1
matrilin 3: NM_002381.2
solute carrier family 14 (urea transporter), member 1 (Kidd blood group): U35735.1
interleukin 7 receptor: M29696.1
MAP kinase kinase 6 (MKK6), mitogen-activated protein kinase kinase 6: U39656.1
protocadherin 17: AF029343.1
granulysin: NM_006433.2
interferon-stimulated protein, 15 kDa (ISG15): M13755.1
cadherin 5, type 2, VE-cadherin (vascularepithelium): U84722.1
thrombomodulin: M16552.1
interferon, alpha-inducible protein 27: NM_005532.1
wherein each oligo- or polynucleotide probe is obtainable on the basis of structural information mediated by the sequence data provided by the respective accession number set out for each candidate gene.

For example, suitable sequences which may be used for oligonucleotide design may be downloaded from the NCBI GenBank (http://www.ncbi.nlm.gov/GenBank/index.html) using the Accession number listed behind the gene name.
All accession numbers give access to mRNA sequences; since the cDNA of the patients is reverse transcribed, the oligonucleotide have to sense strand and are therefore identical with the mRNA sequence.
Preferably the pool of polynucleotide sequences or subsequences correspond substantially to the polynucleotide sequences useful in differentiating a normal cell, or a cell from a patient suffering from non-fibrosing, granulomatous lung disease, from a cell from IPF patient.
The invention concerns also a method for detecting differentially expressed polynucleotide sequences which are correlated with IPF, said method comprising: a) obtaining a polynucleotide sample from a patient; and b) reacting the sample polynucleotide obtained in step (a) with a probe immobilized on a solid support wherein said probe comprises any of the polynucleotide sequences of the libraries previously described or an expression product encoded by any of the polynucleotide sequences of said libraries and c) detecting the reaction product of step (b).

The invention relates also to a method for detecting differentially expressed polynucleotide sequences of the invention wherein the amount of reaction product of step (c) is compared to a control sample.

The method for detecting differentially expressed polynucleotide sequences is used for detecting, diagnosing, staging, monitoring, prognosticating, preventing or treating conditions associated with ILD, and namelly idiopathic pulmonary fibrosis IPF, hypersensivity pneumonitis, sarcoidosis, scleroderma, Systemic Lupus Erythematosus, Rheumatoid Arthritis, Churg-Strauss syndrome, Wegener's granulomatosis, and Goodpasture Syndrome.

The method for detecting differentially expressed polynucleotide sequences is particular useful wherein the product encoded by any of the polynucleotide sequences or subsequences is involved in a receptor-ligand reaction on which detection is based.

The invention relates also to a method for screening an anti-ILD agent comprising the method for detecting differentially expressed polynucleotide sequences previously described wherein the sample has been treated with the anti-ILD medicament to be screened.

The invention also relates to a library of polynucleotides comprising a population of polynucleotide sequences overexpressed or underexpressed in cells derived from ILD patients.

A particular embodiment of the invention relates to a polynucleotide library of corresponding substantially to any combination of at least one polynucleotide sequence selected among those included in each one of predefined polynucleotide sequences sets mentioned above.

The invention relates obviously to polynucleotide libraries comprising at least one polynucleotide selected among those included in at least 50%, preferably 75% and more preferably 100% of said predefined sets, allowing to obtain a discriminating gene pattern, namely to distinguish between normal individuals and patients suffering from I LD.

Polynucleotide sequences library useful for the realization of the invention can comprise also any sequence comprised between 3'-end and 5'-end of each polynucleotide sequence sets as defined above, allowing the complete detection of the implicated genes.

The invention relates also to a polynucleotide library useful to differentiate a normal cell from a cell of ILD patients wherein the pool of polynucleotide sequences or subsequences correspond substantially to any combination of at least one polynucleotide sequence selected among those included in each one of predefined polynucleotide sequences sets indicated above, useful in differentiating a normal cell from a cell from ILD patients.

Differences in gene expression are accepted as different when the signals from patient material are at least two fold lower or higher than those from comparative material, be it from healthy volunteers material or from other diseased material. This threshold is commonly accepted for the evaluation of expression arrays.

The invention additionally provides a method for identifying gene expression or genomic DNA of infective agents including bacteria, yeasts, fungi, or viruses as cellular parasites in cells from patients with ILD.

### Examples

Biopsies were taken from patients suffering from ILD after informal consent was given using the surgical method of bronchoscopy. Obtained tissue probes were stored and processed for isolation of total RNA in RNA*later*™ (patent pending), an aqueous, nontoxic tissue storage reagent that stabilizes and protects cellular RNA in intact, unfrozen tissue samples.
The dissected tissue (less than 0.5 cm in any one dimension) is submerged in approximately 5 volumes of RNA*later* (e.g., a 0.5 g sample requires about 2.5 ml of RNA*later*) at room temperature. The solution permeates the cells, stabilizing the RNA. Then, RNA is isolated using the one-step RNA isolation methods, such as TRlzol® Reagent (Life Technologies), following the instructions of the supplier and finally eluted in H₂O.

**Preparation of Labeled cRNA and Hybridization to Microarrays.** Double-stranded cDNA was synthesized out of the isolated patients RNA samples using a cDNA synthesis kit (Superscript; Life Technologies) employing oligo(dT) priming. The resulting cDNAwas used for *in vitro* transcription (Ambion T7 Megascript system) in the presence of biotin-11-CTP and biotin-16-UTP (Enzo Diagnostics). A total of 25-50 µg of the cRNA product in buffer [40 mM Tris·acetate (pH 8.1)/100 mM potassium acetate/30 mM magnesium acetate] was fragmented at 94°C for 35 min. It was then used as a hybridization probe from each patient for hybridization as recommended (Affymetrix, Santa Clara, CA).

Aliquots of the hybridization patients cRNA mixtures (10 µg cRNA in 200 µl hybridization mix) were hybridized to a Human Genome U133A Array. Each array was washed and scanned (Hewlett Packard, GeneArray scanner G2500A) according to procedures developed by manufacturer (Affymetrix).
**Analysis of GENECHIP Data**. Scanned output files were visually inspected for hybridization artifacts and then analyzed with GENECHIP 3.1 software (Affymetrix).
The expression analysis files created by GENECHIP 3.1 software were transferred to a database (Microsoft Access) and linked to Internet genome databases (e.g., NHLBI, or Swiss Prot).

## Claims

1. A polynucleotide library useful in the molecular characterization of an interstitial lung disease (ILD), said library comprising a pool of polynucleotide sequences or subsequences thereof wherein said sequences or subsequences are either underexpressed or overexpressed in ILD cells.

2. A polynucleotide library from Claim 1 wherein said sequences are useful in classifying good and poor prognosis in ILD patients.

3. A polynucleotide library according to Claim 1 wherein said polynucleotide sequences or subsequences thereof of said pool correspond to any combination of at least one polynucleotide selected among those included in at least 50%, preferably 75% and more preferably 100% of the predefined sets.

4. A polynucleotide library according to Claim 1 wherein said polynucleotide sequences or subsequences thereof of said pool correspond to any combination of at least one polynucleotide selected among those included in at least 50%, preferably 75% and more preferably 100% of the predefined sets for diagnosing Idiopathic pulmonary fibrosis.

5. A polynucleotide library according to any of Claims 1 to 4 wherein said polynucleotides are immobilized on a solid support in order to form a polynucleotide array.

6. A polynucleotide library according to Claim 5 wherein the support is selected from the group comprising a nylon membrane, nitrocellulose membrane, glass slide, glass beads, membranes on glass support or a silicon chip.

7. Method for diagnosing the presence of or predisposition for ILD using expression profiling images comprising the steps of:
(a) isolation of material from an individual clinically diagnosed for ILD, or of familiar predisposition for an ILD,
(b) providing molecular probes as ligands selected from the group consisting of mRNA, polynucleotides, oligonucleotides, cDNAs, proteins or functional fragments thereof, isolated or generated from said individuals
(c) contacting said molecular probes as ligands with a set of immobilized receptors selected from the group consisting of polynucleotides, oligonucleotide or cDNA probes and antibodies or functional fragments thereof, specifically representing a respective set of genes involved with the presence of or predisposition for ILD to be molecularly diagnosed, and qualitatively and quantitatively detecting the presence of bound ligand/receptor complexes to obtain an expression profiling image being representative for the current status of the individual to be diagnosed,
(d) comparing the expression profiling image obtained in step (c) with the expression profiling image(s) of normal individuals and/or with the expression profiling images(s) of individuals having a predisposition for or suffering from ILD to be diagnosed, and
(e) excluding or diagnosing suspected ILD or a predisposition therefore on the basis of the results of the comparison obtained in step (d).

8. Method according to any one of the preceding claims, wherein the ILD or predisposition(s) to be diagnosed is/are selected from the group consisting of idiopathic pulmonary fibrosis (IPF), hypersensivity pneumonitis, sarcoidosis, scleroderma, Systemic Lupus Erythematosus, Rheumatoid Arthritis, Churg-Strauss syndrome, Wegener's granulomatosis, and Goodpasture Syndrome.

9. Method according to any of the preceding claims, wherein in step (c) said set of receptors specifically representing a respective set of genes is selected from the group consisting of genes coding for:
PTH-responsive osteosarcoma B1 protein, AF095771.1
matrix associated, actin dependent regulator of chromatin, subfamily f, member 1, AF231056.1
deleted in lung and esophageal cancer 1 (DLEC1), NM_007337.1
major histocompatibility complex, class II, DQ beta 1, AW276186
SB classII histocompatibility antigen alpha-chain, AI128225
mucin 4, tracheobronchial, AJ242547.1
forkhead box J1 (FOXJ1), U69537.1
hypothetical protein FLJ21616, NM_024567.1
neuronal specific transcription factor DAT1, AF258348.1
hematopoietic PBX-interacting protein, BF344265
proline oxidase homolog, AA074145
mucin 5, subtype B, tracheobronchial, AI697108
golgi membrane protein GP73, AF236056.1
ATP citrate lyase, U 18197.1
NG22 protein, NM_025257.1
cDNA DKFZp434A2322, AL137706.1
hepatocyte nuclear factor 3, alpha, U39840.1
major histocompatibility complex, class II, DQ alpha 1, X00452.1
myosin regulatory light chain 2, smooth muscle isoform, J02854.1
plexin B1, AV693216
pyruvate kinase, muscle, BC000481.1
tetraspanin TM4-C, AF133425.1
insulin-like growth factor binding protein 2 (36kD), BC004312.1
FLJ13945 fis, clone Y79AA1000969, AU160041
hypothetical protein DKFZp586M1120, NM_031294.1
CD24 signal transducer, L33930
hypothetical protein FLJ23571, NM_025111.1
glutathione S-transferase M2 (muscle), M63509.1
cadherin 1, type 1, E-cadherin (epithelial), L08599.1
NTT5 protein, AF265578.1
lipocalin 2 (oncogene 24p3), NM_005564.1
myotonic dystrophy kinase (DM kinase), L08835
uncoupling protein 2 (mitochondrial, proton carrier), U76367.1
dynein intermediate chain 2, NM_023036.1
discoidin receptor tyrosine kinase isoform b, discoidin domain receptor family, member 1, NM_001954.2
sperm associated antigen 6, AF079363.1
hypothetical protein FLJ23049, NM_024687.1
nasopharyngeal epithelium specific protein 1, AF094758.1
nuclear receptor subfamily 4, group A, member 2, NM_006186.1
hypothetical protein FLJ22215, BC003543.1
non-specific cross reacting antigen, M18728.1
amylase, alpha 1A; salivary (AMY1A), NM_004038.1
carcinoembryonic antigen-related cell adhesion molecule 6 (non-specific cross reacting antigen), BC005008.1
glutathione S-transferase subunit 4 (EC 2.5.1.18), X08020.1
SH3-containing protein SH3GLB2, AF257319.1
KDEL (Lys-Asp-Glu-Leu) endoplasmic reticulum protein retention receptor 1, NM_006801.1
anterior gradient 2 (Xenepus laevis) homolog, AF038451.1
sv7-MUC4 apomucin, mucin 4, tracheobronchial, AJ242547.1
stratifin, BC000329.1
connective tissue growth factor, M92934.1
cytochrome P450-IIB (hIIB3), M29873.1
filamin A, alpha (actin-binding protein-280), AW051856
membrane glycoprotein LIG-1, AB050468.1
E74-like factor 3 (ets domain transcription factor, epithelial-specific ), U73844.1
elastin (supravalvular aortic stenosis, Williams-Beuren syndrome), M36860.1
ephrin receptor EPHA3, AF213459.1
fibrillin 1 (Marfan syndrome), L13923.1
discs, large (Drosophila) homolog 1, U13896.1
lysyl oxidase-like 1 (LOXL1), L21186.1
calumenin, U67280.1
male germ cell-associated kinase, NM_005906.2
plectin 1, intermediate filament binding protein, 500kD, Z54367
peroxisome biogenesis factor 1, AF026086.1
mast cell tryptase beta III, AF099143
ataxia-telangiectasia group D-associated protein, AF230388.1
hypothetical protein FLJ10921, NM_018272.1
biglycan, BC002416.1
BLu protein, AC002481
CGI-92 protein, AF151850.1
adaptor-related protein complex 1, mu 2 subunit, BC003387.1
keratin 15, BC002641.1
B7 protein, U72508.1
S100 calcium-binding protein A2, BC002829.1
MUF1 protein, BC004953.1 cholesterol 25-hydroxylase, AF059214.1
cytidine monophosphate-N-acetylneuraminic acid hydroxylase (CMP-N-acetylneuraminate monooxygenase), AF074480.1
neuropilin 2, AF022859.1
Fas-interacting serinethreonine kinase 3, homeodomain-interacting protein kinase 3, AF305239.1
a disintegrin and metalloproteinase domain 28 (ADAM28), transcript variant 2, AF137334.1
cDNA DKFZp434A119 , AW663632
complement component 6, J05064.1
cytokeratin 17, Z19574
wingless-type MMTV integration site family, member 5A, AI968085
matrix metalloproteinase 7 (matrilysin, uterine), BC003635.1
leiomodin 1 (smooth muscle), NM_012134.1
Cip1-interacting zinc finger protein, AB030835.1
cyclin-dependent kinase inhibitor 1A (p21, Cip1), BC000275.1
integrin, alpha 7, AF032108.1
DKFZP586G011 protein, BG289527
fatty acid binding protein 6, ileal (gastrotropin), U 19869.1
glutathione S-transferase M4, M96234.1
Ras-related associated with diabetes, L24564.1
claudin 3, AB000714.1
matrix metalloproteinase 10 (stromelysin 2), BC002591.1
fibulin 2, NM_001998.1
serine threonine kinase 11 (STK11), AF035625
sorting nexin 10 (SNX10), AF121860.1
phospholipase A2, group IIA (platelets, synovial fluid), M22430.1
hemoglobin alpha-1 globin chain (HBA1), AF349571.1
macrophage scavenger receptor 1, AI299239
surfactant, pulmonary-associated protein C, BC005913.1
disintegrin protease (M12.219), NM_014479.1
retinol-binding protein 4, interstitial, AF119868.1
major histocompatibility complex, class II, DR beta 3, M27635.1
adipose differentiation-related protein, BC005127.1
hemoglobin, delta, NM_000519.2
beta-2-microglobulin, AW188940
hemoglobin, alpha 2, BC005931.1
protein C receptor, endothelial (EPCR), L35545.1
thymosin, beta 10, M92381.1
apolipoprotein C-I, W79394
hypothetical protein, AL133067.1
proteoglycan 4, (megakaryocyte stimulating factor, articular superficial zone protein), U70136.1
tetranectin (plasminogen-binding protein), NM_003278.1
platelet factor 4, M25897.1
tissue factor pathway inhibitor 2, BC005330.1
fatty acid binding protein 4, adipocyte, BC003672.1
cathepsin B (CTSB), M14221.1
transmembrane 4 superfamily member 1, M90657.1
MHC class I HLA-B51 major histocompatibility complex, class I, E, M31183.1
endothelial PAS domain protein 1, U51626.1
Apo-2 ligand tumor necrosis factor (ligand) superfamily, member 10, U37518.1
haptoglobin-related protein, NM_020995.1
Rho guanine exchange factor (GEF) 12, AF119898.1
major histocompatibility complex, class II, DR beta 5, M11867.1
interferon, gamma-inducible protein 30, AF097362.1
ferritin, light polypeptide, BG537190
prosaposin (variant Gaucher disease and variant metachromatic leukodystrophy), BC004275.1
calcium-binding protein A4 (calvasculin, metastasin,), NM_002961.2
hemoglobin, beta, M25079.1
CDW52 antigen (CAMPATH-1 antigen), BC000644.1
aldehyde dehydrogenase 1 family, member A2, AB015226.1
cathepsin Z, AF032906.1
MHC HLA-B39 major histocompatibility complex, class I, B, L37880.1
major histocompatibility complex, class II, DQ alpha 1, M33906.1
fibroblast growth factor 9 (glia-activating factor), D14838.1
hemoglobin, alpha 2, AF097635.1
transferrin receptor (p90, CD71), BC001188.1
complement component 3 (C3), K02765.1
cDNA DKFZp564D066, AL050025.1
complement component 1, q subcomponent, beta polypeptide, NM_000491.2
small inducible cytokine subfamily A (Cys-Cys), member 18, pulmonary and activation-regulated, AB000221.1
reticulon 1, L10333.1
major histocompatibility complex, class II, DR beta 1, M33600.1
haptoglobin, L29394.1
acid phosphatase 5, tartrate resistant, J04430.1
cytochrome P450, subfamily XXVIIA (steroid 27-hydroxylase, cerebrotendinous xanthomatosis), polypeptide 1, M62401.1
CD36 antigen (collagen type I receptor, thrombospondin receptor), M24795.1
calbindin 2, (29kD, calretinin), NM_001740.2
alpha-2-HS-glycoprotein, BG538564
cDNA DKFZp564A132, AL049963.1
fibronectin 1, AF130095.1
phosphodiesterase 4C, cAMP-specific, NM_000923.1
transcription factor 7 (T-cell specific, HMG-box), NM_003202.1
found in inflammatory zone 3 (FIZZ3), AF323081.1
claudin 15, NM_014343.1
carboxypeptidase B1 (tissue), M81057.1
hypothetical protein FLJ14054, NM_024563.1
bone marrow stromal cell antigen 1, D21878.1
interleukin 7 receptor, M29696.1
procollagen C-endopeptidase enhancer 2, AF098269.1
calcium-binding protein A8 (calgranulin A), AW238654
cDNA DKFZp564D193, AL049252.1
major histocompatibility complex, class II, DP beta 1, J03041.1
human leukocyte antigen C alpha chain, major histocompatibility complex, class I, C, AK024836.1
BCM-like membrane protein precursor, AF144235.1
CD14 antigen, M86511.1
pulmonary surfactant protein (SP5), J03553
signal transducer and activator of transcription 1, 91 kD, BC002704.1
Wilms tumor 1 (WT1), transcript variant D, NM_024424.1
annexin A8, BC004376.1
macrophage receptor with collagenous structure, MARCO, AF035819.1
surfactant, pulmonary-associated protein A2, NM_006926.1
solute carrier family 6 (neurotransmitter transporter, serotonin), member 4, L05568.1
chitinase 1 (chitotriosidase), U29615.1
lung type-I cell membrane-associated glycoprotein, AU154455
fibronectin leucine rich transmembrane protein 2, AB007865.1
gamma-aminobutyric acid (GABA) A receptor, alpha 5, BF966183
hypothetical protein FLJ12983, NM_024856.1
sialophorin (gpL115, leukosialin, CD43), J04536.1
cerebellar degeneration-related protein (34kD), M16965.1
hydroxyacid oxidase 2 (long chain), hydroxy-delta-5-steroid dehydrogenase (3 beta-and steroid delta-isomerase 2), AL359553
eukaryotic translation initiation factor 1A: AF000987.1
DEADH (Asp-Glu-Ala-AspHis) box polypeptide: AF000985.1
ribosomal protein S4: AF116711.1
ubiquitin specific protease 9: AF000986.2
SMC (mouse) homolog: U52191.1
myelin basic protein: L18865.1
S100 calcium-binding protein: NM_005980.1
Jagged2 (JAG2): AF003521.1
latent transforming growth factor beta binding protein 4: NM_003573.1
microtubule-associated protein, RPEB family, member 3: AB025186.1
Unknown (protein for MGC:2854): BC003629.1
clone=IMAGE-2406340: AI830563
AQP3 gene for aquaporine 3 (water channel): AB001325
cDNA DKFZp434A119
fenestrated-endothelial linked structure protein (FELS), PV1 protein (PLVAP): AF326591.1
LUNX protein; PLUNC (palate lung and nasal epithelium clone); tracheal epithelium enriched protein (LOC51297): AB024937.1
RAB, member of RAS oncogene family-like 2A: AF095350.1
chromosome 11 open reading frame 16: NM_020643.1
hypothetical protein FLJ23049: NM_024687.1
hypothetical protein FLJ11767: NM_024593.1
dynein, axonemal, intermediate polypeptide: AF091619.1
brain specific protein (LOC51673): AF132972.1
MUC4 apomucin, mucin 4, tracheobronchial: AJ242547.1
myotonin protein kinase (DM): M87313.1
cytokeratin 4: X07695.1
KIAA0362 gene, MCF.2 cell line derived transforming sequence-like: AB002360.1
cytokeratin 17: Z19574
LIM domain protein: BC003096.1
E74-like factor 3 (ets domain transcription factor, epithelial-specific ) : U73844.1
fatty acid binding protein 6, ileal (gastrotropin): U19869.1
sperm associated antigen 6: AF079363.1
eyes absent (Drosophila) homolog 2: U71207.1
phosphatidic acid phosphatase type 2C: BC002806.1
epoxide hydrolase 2, cytoplasmic: AF233334.1
tubulin, beta, 2: BC002783.1
heat shock 105kD: D86956.1
villin 2 (ezrin): J05021.1
a disintegrin and metalloproteinase domain 28 (ADAM28), transcript variant 3: AF137335.1
deleted in lung and esophageal cancer 1: NM_007337.1
arachidonate 15-lipoxygenase: NM_001140.1
UDP glycosyltransferase 1 family, polypeptide A1: M57899.1
hypothetical protein PRO2834: AF119903.1
lectin, galactoside-binding, soluble, 7 (galectin 7): L07769.1
B7 protein: U72508.1
ephrin receptor EPHA3: AF213459.1
forkhead box J1: U69537.1
BLu protein: U70824.1
aldehyde dehydrogenase 3 family, member A1: BC004370.1
NG22 protein: NM_025257.1
small inducible cytokine subfamily A (Cys-Cys), member 14: NM_004166.1
cysteine-rich protein 1 (intestinal): BC002738.1
putative GTP-binding protein similar to RAYRAB1C: BC000566.1
integrin, beta 4: NM_000213.1
serine (or cysteine) proteinase inhibitor, clade B (ovalbumin), member 5 (SERPINB5): U04313.1
Ras-related associated with diabetes: L24564.1
hepatic leukemia factor: M95585.1
keratin 15: BC002641.1
nuclear receptor subfamily 4, group A, member 2: NM_006186.1
sialyltransferase: U14550.1
glutathione S-transferase M2 (muscle): M63509.1
hypothetical protein FLJ13110: NM_022912.1
S100 calcium-binding protein A2: NM_005978.2
collagen, type VII, alpha 1 (epidermolysis bullosa, dystrophic, dominant and recessive) : L02870.1
claudin 3: AB000714.1
insulin-like growth factor binding protein 6: BC003507.1
fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome): M80634.1
insulin-like growth factor 1 receptor: NM_000875.2
insulin-like growth factor binding protein 2 (36kD): M35410.1
ataxia-telangiectasia group D-associated protein: AF230388.1
keratin 5 (epidermolysis bullosa simplex, Dowling-MearaKobnerWeber-Cockayne types): M21389.1
Duffy blood group: U01839.1
CLONE=IMAGE:1032795=Hs.83623 nuclear receptor subfamily 1, group I, member 3
small inducible cytokine subfamily C, member 2: NM_003175.1
hypothetical protein similar to swine acylneuraminate lyase: NM_030769.1
fibrinogen, gamma polypeptide: AF118092.1
thrombospondin 1: NM_003246.1
SAM domain, SH3 domain and nuclear localisation signals, 1: AF222927.1
chitinase 3-like 1 (cartilage glycoprotein-39): M80927.1
cathepsin Z: AF032906.1
CLONE=IMAGE:3579023 collagen, type XIV, alpha 1 (undulin)
chloride intracellular channel 2: NM_001289.2
monokine induced by gamma interferon: NM_002416.1
KIAA0433 protein: NM_015216.1
tumor necrosis factor, alpha-induced protein 6: NM_007115.1
signal transducer and activator of transcription 1, 91 kD: BC002704.1
thrombospondin 2: L12350.1
collagen, type IV, alpha 3 (Goodpasture antigen): NM_000091.1
integrin, beta-like 1 (with EGF-like repeat domains): AF072752.1
diubiquitin: NM_006398.1
protease, cysteine, 1 (legumain): D55696.1
cytochrome P450, subfamily I (dioxin-inducible), polypeptide 1 (glaucoma 3, primary infantile): U03688.1
integrin, alpha 1: X68742.1
GABA-B receptor, G protein-coupled receptor 51: AF056085.1
KIAA1199 protein: AB033025.1
collagen, type V, alpha 2: NM_000393.1
interleukin 13 receptor, alpha 2: U70981.1
translocase of inner mitochondrial membrane 8 (yeast) homolog A: BC005236.1
steroid sulfatase (microsomal), arylsulfatase C, isozyme S: M16505.1
CLONE=IMAGE:1982571 ATPase, H+ transporting, lysosomal (vacuolar proton pump) 9kD
proteoglycan 4, (megakaryocyte stimulating factor, articular superficial zone protein): U70136.1
nidogen (enactin): M30269.1
KIAA1598 protein: AU157109 vascular cell adhesion molecule 1: M60335.1
guanylate binding protein 1, interferon-inducible, 67kD: BC002666.1
cDNA DKFZp586E1124
apolipoprotein H (beta-2-glycoprotein I): M62839.1
ribosomal protein L37a: BE857772
cDNA DKFZp564A132
cathepsin S: M86553.1
a disintegrin and metalloproteinase domain 9 (meltrin gamma) (ADAM9): U41766.1
zinc finger protein 331: AF272148.1
lysosomal-associated membrane protein 2: J04183.1
carboxypeptidase M: NM_001874.1
collagen, type I, alpha 2: NM_000089.1
P311 protein: U36189.1
KIAA0372 gene product: AB002370.1
Human T cell-specific protein RANTES: M21121
interferon-gamma-inducible indoleamine 2,3-dioxygenase (IDO): M34455.1
hypothetical protein FLJ10430: NM_018092.1
transcription factor ISGF-3: M97935
interleukin 1 receptor-like 1 (IL1RL1): NM_003856.1
putative alpha chemokine (H174), small inducible cytokine subfamily B (Cys-X-Cys), member 11: AF002985.1
small inducible cytokine subfamily B (Cys-X-Cys), member 10: NM_001565.1
mesoderm specific transcript (mouse) homolog: BC002413.1
carboxypeptidase B-like protein: AB011969.1
CD2 antigen (p50), sheep red blood cell receptor: M16445.
interferon, alpha-inducible protein (clone IFI-6-16): NM_022872.1
RAS guanyl releasing protein 1 (calcium and DAG-regulated): AF081195.1
lipase, endothelial: AF118767.1
fatty-acid-Coenzyme A ligase, long-chain 4: NM_022977.1
klotho: AB005142.1
chondroitin sulfate proteoglycan 2 (versican): NM_004385.1
hypothetical protein, expressed in osteoblast: AB000115.1
CD14 antigen: M86511.1
CGI-83 protein: BC000878.1
leucine aminopeptidase: AF061738.1
UDP-Gal:betaGlcNAc beta 1,3-galactosyltransferase, polypeptide 3: AB050855.1
protocadherin alpha 12: AF152308.1
neuroglycan C: AF059274
neuroligin: AI338338
HSPC156 protein: AF161505.1
hypothetical protein FLJ13310: NM_025118.1
eosinophil chemotactic cytokine (TSA1902): AB025008.1
aminopeptidase: AF191545.1
semaphorin sem2: AB029496.1
protocadherin 12: AF231025.1
X transporter protein 3: NM_020208.1
transmembrane 4 superfamily member (tetraspan NET-2): AF124522.1
hypothetical protein FLJ10970: NM_018286.1
perforin 1 (pore forming protein): M28393.1
natural killer cell group 7 sequence: NM_005601.1
hypothetical protein DKFZp761 N09121: BF435376 integrin, alpha 4 (antigen CD49D, alpha 4 subunit of VLA-4 receptor): BG532690
chitinase 3-like 2: U58515.1
FYN oncogene: N20923
relaxin 1 (H1): BC005956.1
hydroxyprostaglandin dehydrogenase 15-(NAD): U63296.1
sulfotransferase family, cytosolic, 1C, member 1: AF186254.1
TGF-b superfamily receptor type I: L17075.1
granzyme B (granzyme 2, cytotoxic T-lymphocyte-associated serine esterase 1): M36118.1
cystatin F (leukocystatin): AF031824.1
regulator of G protein signaling-Z (RGSZ1): AF060877.2
clone MGC:12387: M16942.1
zinc-alpha2-glycoprotein: D90427.1
BCG induced integral membrane protein BIGMo-103: AB040120.1
nectin-like protein 2 (NECL2): AF132811.1
CD209 antigen-like: AB015629.1
solute carrier family 6 (neurotransmitter transporter, serotonin), member 4: L05568.1
MAD (mothers against decapentaplegic, Drosophila) homolog 6: U59914.1
latrophilin: AF104939.1
platelet factor 4: M25897.1
calcitonin receptor-like: L76380.1
matrilin 3: NM_002381.2
solute carrier family 14 (urea transporter), member 1 (Kidd blood group): U35735.1
interleukin 7 receptor: M29696.1
MAP kinase kinase 6 (MKK6), mitogen-activated protein kinase kinase 6: U39656.1
protocadherin 17: AF029343.1
granulysin: NM_006433.2
interferon-stimulated protein, 15 kDa (ISG15): M13755.1
cadherin 5, type 2, VE-cadherin (vascularepithelium): U84722.1
thrombomodulin: M16552.1
interferon, alpha-inducible protein 27: NM_005532.1
transforming growth factoe beta
Interferon gamma
and allelic variants or mutants thereof.

10. Method according to claim 9, wherein said set of receptors is selected from oligonucleotide probes having a sequence defined by, or correlated to, or derived from the group of genes consisting of candidate genes indicated in claim 9, wherein each oligonucleotide probe is obtainable on the basis of structural information mediated by the sequence data provided by the respective accession number set out for each candidate gene.

11. Method according to any one of the preceding claims, wherein in step (c) said immobilized receptors are located on a surface.

12. A method for detecting differentially expressed polynucleotide sequences according to any preceding claims wherein said polynucleotide sample is labelled before its reaction step.

13. A method for detecting differentially expressed polynucleotide sequences according to any preceding claim wherein the label of the polynucleotide sample is selected from the group consisting of radioactive, colorimetric, enzymatic, molecular amplification, bioluminescent or fluorescent labels.

14. A method for detecting differentially expressed polynucleotide sequences according to Claim 9 wherein the polynucleotide sample is cDNA, RNA or mRNA.

15. A method for detecting differentially expressed polynucleotide sequences according to Claim 9 wherein mRNA is isolated from said polynucleotide sample and cDNA is obtained by reverse transcription of said mRNA.

16. A method for detecting differentially expressed polynucleotide sequences according to Claims 14 to 15 wherein said reaction step is performed by hybridising the polynucleotide sample with the probe.

17. A method for detecting differentially expressed polynucleotide sequences according to Claims 14 to 16 wherein the product encoded by any of the polynucleotide sequences or polynucleotide sequences sets is involved in a receptor-ligand reaction on which detection is based.

18. A method for detecting gene expression and /or genomic DNA of infective agents including bacteria, yeasts, fungi, or viruses as cellular parasites in cells from patients with ILD.
